# EUROPÄISCHE PATENTANMELDUNG

(11) **EP 3 501 437 A1**
(43) Veröffentlichungstag der Anmeldung: **26.06.2019**
(21) Anmeldenummer: 17209169.6
(22) Anmeldetag: 20.12.2017
(51) Int. Cl.: A61B 17/80, A61B 17/86

(54) **KNOCHENPLATTE UND VERFAHREN ZUM HERSTELLEN EINER KNOCHENPLATTE**

(71) Anmelder: Wolter, Dietmar, 22955 Hoisdorf (DE)
(72) Erfinder: Wolter, Dietmar, 22955 Hoisdorf (DE)
(74) Vertreter: Glawe, Delfs, Moll

(57) **Zusammenfassung**

Verfahren zum Herstellen einer Knochenplatte, bei dem ein Inlay (19) in eine Ausnehmung (33) einer tragenden Struktur (20) eingesetzt wird, so dass eine Mantelfläche (22) des Inlays (19) an einer Innenmantelfläche (23) der Ausnehmung (33) anliegt. Das Inlay (19) und die tragende Struktur (20) werden mit einer Schweißnaht (21) verbunden, so dass die Eindringtiefe der Schweißnaht (21) kleiner ist als die Materialstärke im Bereich der Schweißnaht (21). Die Erfindung betrifft außerdem eine zugehörige Knochenplatte.

## Beschreibung

Die Erfindung betrifft eine Knochenplatte sowie ein Verfahren zum Herstellen einer Knochenplatte.

Knochenplatten können im Rahmen einer chirurgischen Operation mit einem Knochen verbunden werden. Sie werden beispielsweise verwendet, um einen Knochen nach einem Bruch zu stabilisieren. Dazu wird die Knochenplatte so positioniert, dass sie sich über die Bruchstelle hinweg erstreckt, und dann an den Knochenfragmenten befestigt. Die Bruchstelle wird dadurch ruhig gestellt und der Knochen kann ausheilen. Die Knochenplatte kann auch anderen Zwecken dienen und beispielsweise ein mit einem Knochen zu verbindendes Element einer Endoprothese sein.

Zur Befestigung einer solchen Knochenplatte am Knochen kann eine Knochenschraube verwendet werden, deren Schaft und deren Kopf mit einem Gewinde versehen sind. Der Schaft wird durch ein Durchgangsloch der Knochenplatte hindurchgeführt und in das Knochenmaterial eingedreht, bis der Kopf der Schraube in das Durchgangsloch der Knochenplatte eintritt. Das Gewinde am Kopf der Knochenschraube kommt mit dem das Durchgangsloch umgebenden Material der Knochenplatte in Eingriff, so dass unter Umformung des Materials der Knochenplatte eine Gewindeverbindung gebildet wird. Der Winkel, unter dem die Gewindeverbindung hergestellt wird, kann innerhalb eines vorgegebenen Bereichs frei gewählt werden. Die Verbindung ist also multidirektional winkelstabil, so dass der Chirurg den Eindrehwinkel während der Operation wählen kann, wobei trotzdem eine winkelstabile Verbindung zwischen der Knochenschraube und der Knochenplatte entsteht.

Für die Verwendung einer solchen Knochenplatte ist es von Vorteil, wenn die Knochenplatte im Bereich des Durchgangslochs aus einem Material besteht, das sich gut umformen lässt. Es ist bekannt, zu diesem Zweck ein Inlay in eine tragende Struktur der Knochenplatte einzusetzen.

Ein solches Inlay kann beispielsweise durch Schweißen mit der tragenden Struktur der Knochenplatte verbunden werden, DE 196 29 011 A1, EP 2 801 330 A1, WO 2010/115458 A1. Beim Schweißen wird Wärme in das Material eingebracht, woraus sich unerwünschte Auswirkungen auf die Materialstruktur ergeben können.

Der Erfindung liegt die Aufgabe zu Grunde, eine Knochenplatte sowie ein Verfahren zum Herstellen einer Knochenplatte vorzustellen, bei dem die Wahrscheinlichkeit von unerwünschten Veränderungen der inneren Struktur der Knochenplatte vermindert ist. Ausgehend vom genannten Stand der Technik wird die Aufgabe gelöst mit den Merkmalen der unabhängigen Ansprüche. Vorteilhafte Ausführungsformen sind in den Unteransprüchen angegeben.

Bei dem erfindungsgemäßen Verfahren wird ein Inlay in eine Ausnehmung einer tragenden Struktur eingesetzt, so dass eine Mantelfläche des Inlays an einer Innenmantelfläche der Ausnehmung anliegt. Das Inlay und die tragende Struktur werden durch eine Schweißnaht miteinander verbunden, wobei die Eindringtiefe der Schweißnaht in einer Ausführungsform der Erfindung kleiner ist als die Materialstärke im Bereich der Schweißnaht.

Indem die Schweißnaht sich nur über einen Teil der Materialstärke erstreckt, kann der Wärmeeintrag vermindert werden, der beim Erzeugen der Schweißnaht auf die Knochenplatte wirkt. Durch den verminderten Wärmeeintrag in das Material der Knochenplatte kann eine Veränderung der Materialstruktur vermieden werden. Insbesondere kann in dem Bereich des Inlays, in dem der Schraubenkopf eingreift, eine auf gute Verformbarkeit ausgelegte Materialstruktur des Inlays erhalten bleiben. Das Material des Inlays kann mit dem Eindrehen der Knochenschraube unter geringem Kraftaufwand verformt werden.

Die tragende Struktur kann eine Ausnehmung mit einer zu der Mantelfläche des Inlays passenden Innenmantelfläche haben. Wenn das Inlay in die tragende Struktur eingesetzt wird, können die Mantelfläche des Inlays und die Innenmantelfläche der tragenden Struktur entlang einer Kontaktfläche flächig aufeinander liegen. Die Materialstärke im Bereich der Schweißnaht entspricht der Höhe der Kontaktfläche, also derjenigen Fläche, über die die Mantelfläche des Inlays und die Innenmantelfläche der tragenden Struktur flächig aufeinander liegen. Als Höhe wird die Richtung bezeichnet, die sich senkrecht zu der Umfangsrichtung der Mantelfläche bzw. der Innenmantelfläche erstreckt.

Wenn die Schweißnaht sich nur über einen Teil der Materialstärke erstreckt, gibt es auf der der Schweißnaht gegenüberliegenden Seite einen Übergangsbereich zwischen dem Inlay und der tragenden Struktur, an dem die beiden Teile nur aneinander liegen, aber nicht miteinander verschweißt sind. Bei dem erfindungsgemäßen Verfahren kann eine mechanische Bearbeitung des Übergangsbereichs erfolgen. Durch die mechanische Bearbeitung kann ein eventueller Spalt zwischen dem Inlay und der tragenden Struktur verschlossen werden, beispielsweise indem der Übergangsbereich übergefräst wird. Es kann eine geschlossene Oberfläche erzeugt werden, so dass ein Eindringen von Verunreinigungen verhindert wird.

Die mechanische Bearbeitung kann so erfolgen, dass in dem Übergangsbereich Material von dem Inlay und/oder von der tragenden Struktur abgetragen wird, so dass die Materialstärke im Bereich der Kontaktfläche vermindert wird. Insbesondere ist es möglich, in dem Übergangsbereich so viel Material abzutragen, dass der Höhenabschnitt des Inlays und/oder der tragenden Struktur vollständig entfernt wird, in dem das Inlay und die tragende Struktur aneinander liegen, jedoch nicht miteinander verschweißt sind. Wenn mit der mechanischen Bearbeitung bis zu der Schweißnaht vorgedrungen wird, so kann eine Knochenplatte erzeugt werden, bei der das Inlay und die tragende Struktur über den gesamten noch verbleibenden Höhenabschnitt durchgehend miteinander verschweißt sind.

Bei dem Verfahren kann ein Inlay verwendet werden, das bereits vor dem Einsetzen in die tragende Struktur mit einem Durchgangsloch versehen ist. Bei der Verwendung eines solchen Inlays kann die Möglichkeit bestehen, auf eine weitere Bearbeitung des Durchgangslochs nach dem Verschweißen des Inlays mit der tragenden Struktur zu verzichten. Insbesondere kann auf eine nachfolgende Bearbeitung verzichtet werden, wenn in dem Durchgangsloch bereits eine von der Lochwand nach innen vorspringende Materiallippe ausgebildet ist. Möglich ist auch, eine solche Materiallippe in einem vorgefertigten Durchgangsloch nachträglich zu erzeugen.

Alternativ kann ein Inlay in die tragende Struktur eingesetzt werden, das zu diesem Zeitpunkt noch kein Durchgangsloch aufweist. Wenn das Inlay zu dem Zeitpunkt, zu dem die Schweißnaht erzeugt wird, einen Vollkörper ohne Durchgangsloch bildet, kann die durch das Schweißen erzeugte Wärme sich besser verteilen, so dass das Risiko von Strukturveränderungen in dem Material weiter sinkt.

Nachdem das Inlay durch Schweißen mit der Tragestruktur verbunden wurde, kann ein Durchgangsloch in dem Inlay erzeugt werden. Das Durchgangsloch kann so erzeugt werden, dass von der Lochwand eine Materiallippe nach innen vorspringt.

Die Knochenplatte kann eine dem Knochen zugewandte Unterseite und eine von dem Knochen abgebrannte Oberseite aufweisen, wenn die Knochenplatte bestimmungsgemäß mit einem Knochen verbunden ist. Ein in der Knochenplatte ausgebildetes Durchgangsloch kann sich zwischen der Unterseite und der Oberseite erstrecken.

Das erfindungsgemäße Verfahren kann so durchgeführt werden, dass die Schweißnaht von der Unterseite der Knochenplatte ausgeführt ist. Der Übergangsbereich, in dem das Inlay und die tragende Struktur aneinander liegen, ohne miteinander verschweißt zu sein, kann in Richtung der Oberseite der Knochenplatte ausgerichtet sein. Die Schweißnaht kann beispielsweise durch Laserschweißen erzeugt sein.

Die Mantelfläche des Inlays kann zylinderförmig oder konusförmig sein. Möglich ist auch, dass der zylinderförmige Bereich und/oder der konusförmig Bereich sich nicht über die gesamte Materialstärke, sondern nur über einen Höhenabschnitt der Materialstärke erstreckt. Insbesondere kann das Inlay einen ersten Höhenabschnitt aufweisen, in dem die Mantelfläche zylinderförmig ist und einen zweiten Höhenabschnitt aufweisen, in dem die Mantelfläche konusförmig ist. Der konusförmige Höhenabschnitt kann sich zu dem zylinderförmigen Höhenabschnitt hin verjüngen. Von der Erfindung umfasst sind auch Mantelflächen, die nicht rotationssymmetrisch sind.

Wenn das Inlay konusförmig ausgebildet ist oder einen konusförmigen Höhenabschnitt aufweist, so können der breitere Abschnitt des Konus zur Unterseite der Knochenplatte und der schmalere Abschnitt des Konus zur Oberseite der Knochenplatte weisen. Die Differenz zwischen dem größten Durchmesser des Konus und dem kleinsten Durchmesser des Konus kann zwischen 0,01 mm und 0,1 mm, vorzugsweise zwischen 0,02 mm und 0,05 mm liegen.

Um das Inlay und die tragende Struktur vor dem Schweißen relativ zueinander zu positionieren, kann ein die richtige Position definierender Anschlag verwendet werden. Der Anschlag kann ein Anschlag innerhalb der Knochenplatte sein, wie er sich beispielsweise daraus ergeben kann, dass eine konusförmige Außenfläche eines Inlays an einer konusförmigen Fläche der tragenden Struktur anliegt. Möglich ist auch ein Anschlag außerhalb der Knochenplatte. Beispielsweise kann die Kombination aus der tragenden Struktur und dem Inlay kann dann auf einen Anschlag aufgelegt werden, so dass die Unterseite zum Herstellen der Schweißnaht gut zugänglich ist. Dafür kann es von Vorteil sein, wenn die Oberseite des in die tragende Struktur eingesetzten Inlays bündig mit der Oberseite der tragenden Struktur abschließt.

Das in die tragende Struktur eingesetzte Inlay kann die gleiche Dicke haben wie die tragende Struktur. In einer Ausführungsform erstreckt die Kontaktfläche zwischen dem Inlay und der tragenden Struktur sich über die gesamte Dicke des Inlays.

Das Inlay kann der Oberseite und der Unterseite bündig mit der tragenden Struktur abschließen. Möglich ist auch, dass die Kontaktfläche sich nur über einen Teil der Dicke des Inlays erstreckt, beispielsweise über mindestens 50 %, vorzugsweise über mindestens 70 % der Dicke des Inlays. Die Dicke bezeichnet den Abstand zwischen der Oberseite und der Unterseite der Knochenplatte in dem betreffenden Bereich.

Von der Erfindung umfasst sind auch Ausführungsformen, bei denen das Inlay an der Unterseite gegenüber der tragenden Struktur vorspringt, wodurch die zur Wärmeableitung zur Verfügung stehende Oberfläche des Inlays vergrößert werden kann. Wenn die Unterseite im eingesetzten Zustand der Knochenplatte auf dem Knochen aufliegt, übt die tragende Struktur in der Umgebung des Inlays einen verminderten Druck auf das Knochenmaterial aus, so dass die Versorgung des Knochens in geringerem Maße beeinträchtigt wird. An der Oberseite der Knochenplatte kann das Inlay bündig mit der tragenden Struktur abschließen. Von der Erfindung umfasst sind auch Ausführungsformen, bei denen die Dicke des Inlays geringer ist als die Dicke der tragenden Struktur.

Das Inlay kann eine Materiallippe umfassen, die in Richtung des Zentrums des Durchgangslochs vorspringt. Die Materiallippe kann sich in Umfangsrichtung des Durchgangslochs erstrecken. Die Materiallippe kann sich über einen Teil des Umfangs oder über den gesamten Umfang erstrecken. Die Materiallippe kann den Bereich des Inlays bilden, der mit dem Eindrehen der Knochenschraube umgeformt wird.

Bezogen auf die Dicke des Inlays kann die Materiallippe innerhalb einer Hälfte des Inlays angeordnet sein. Die anderen 50 % der Dicke des Inlays sind dann frei von der Materiallippe.

Insbesondere ist es möglich, dass bezogen auf die Dicke des Inlays die Materiallippe in einem zu der Oberfläche benachbarten Drittel angeordnet ist. Ein an die andere Oberfläche angrenzender Abschnitt von zwei Drittel ist demnach frei von der Materiallippe.

Die Materiallippe kann in einem Höhenabschnitt des Inlays angeordnet sein, der kleiner ist als 50%, vorzugsweise kleiner ist als 30% der Dicke des Inlays. Die Materiallippe kann in einem zu der Unterseite der Knochenplatte benachbarten Höhenabschnitt des Inlays angeordnet sein. Oberhalb der Materiallippe verbleibt dann Raum, innerhalb dessen der Kopf der Knochenschraube aufgenommen werden kann.

Wenn durch die Erfindung negative Einflüsse auf die Materialstruktur vermieden werden, können der zum Umformen des Inlays und der zum Erzeugen der Gewindeverbindung erforderliche Kraftaufwand gering gehalten werden. Eine Verminderung dieses Kraftaufwands ist insbesondere bei größeren Knochenplatten von Interesse, bei denen hohe Kräfte wirken. Die Knochenschrauben und die Knochenplatte sind dann größer dimensioniert und es wird mehr Material des Inlays umgeformt, um eine hinreichend stabile Gewindeverbindung zwischen dem Kopf der Knochenschraube und der Knochenplatte zu erreichen. Die Erfindung kann vor diesem Hintergrund insbesondere bei dickeren Knochenplatten zur Anwendung kommen. Beispielsweise kann die Dicke der Knochenplatte größer als 2 mm sein und insbesondere zwischen 3 mm und 5 mm liegen. Als Dicke der Knochenplatte wird der Abstand zwischen der Oberseite und Unterseite bezeichnet, den die tragende Struktur im Bereich des Übergangs zu dem Inlay hat.

Die erfindungsgemäße Knochenplatten kann für einen großen Röhrenknochen des menschlichen Körpers bestimmt sind, weil diese Knochenplatten großen Hebelkräften ausgesetzt sein können. Knochenplatten für diesen Anwendungsbereich haben eine größere Dicke als andere Knochenplatten. Beispielsweise kann die Knochenplatte für einen Oberschenkelknochen bestimmt sein.

Die Schweißnaht kann sich von einer Oberfläche der Knochenplatte in die Tiefe des Materials erstrecken. Die Schweißnaht erstreckt sich vorzugsweise über nicht mehr als 80 %, weiter vorzugsweise nicht mehr als 70 %, weiter vorzugsweise nicht mehr als 60 % der Höhe der Kontaktfläche. Beispielweise kann die Schweißnaht um 1 mm bis 3 mm, vorzugsweise um 1,5 mm bis 2,5 mm in das Material der Knochenplatte eindringen.

Um das Umformen zu erleichtern, kann das Material des Inlays weicher sein als das Material der tragenden Struktur. Für die Herstellung der erfindungsgemäßen Knochenplatte sind Titanmaterialien geeignet. Bei unlegiertem Titan (Reintitan) hängt die Härte bekanntlich vom Sauerstoffgehalt ab. Je höher der Sauerstoffgehalt desto größer ist die Härte. Das Inlay kann aus Reintitan geringer Härte hergestellt sein. Es kann beispielsweise Reintitan Grade 0 oder Grade 1 verwendet werden, bei dem der Sauerstoffgehalt beispielsweise kleiner als 0,2 %, vorzugsweise kleiner als 0,1 % sein kann.

Die tragende Struktur der Knochenplatte kann aus Reintitan von größerer Härte gefertigt sein. Beispielsweise kann die tragende Struktur aus Reintitan Grade 4 bestehen. Der Sauerstoffgehalt des Reintitans kann beispielsweise zwischen 0,3 % und 0,4 % liegen. Möglich ist auch, die tragende Struktur aus einer Titanlegierung herzustellen. Titanlegierungen haben regelmäßig eine größere Härte als Reintitan. In Betracht kommt beispielsweise TiAl6V4 oder eine Titan-Niob-Legierung.

Die Knochenplatte kann eine Mehrzahl von Durchgangslöchern aufweisen. Es ist möglich, dass ein Teil der Durchgangslöcher in der tragenden Struktur der Knochenplatte ausgebildet ist. In einer vorteilhaften Ausführungsform sind alle Durchgangslöcher in dem weicheren Inlay-Material angeordnet. Die Knochenplatte kann für jedes Durchgangsloch ein eigenes Inlay aufweisen. Möglich ist auch, dass ein Inlay eine Mehrzahl von Durchgangslöchern aufweist.

Die Erfindung betrifft ferner eine Knochenplatte mit einem Durchgangsloch, das sich zwischen einer Oberseite und einer Unterseite der Knochenplatte erstreckt. Eine tragende Struktur der Knochenplatte kann aus einem ersten Material gebildet sein. Das Durchgangsloch kann in einem Inlay aus einem zweiten Material ausgebildet sein. Die tragende Struktur und das Inlay können durch eine Schweißnaht miteinander verbunden sein. Ein der Schweißnaht gegenüberliegender Übergangsbereich zwischen der tragenden Struktur und dem Inlay kann mechanisch bearbeitet sein.

Die Erfindung betrifft außerdem ein System aus einer solchen Knochenplatte und einer Knochenschraube. Der Kopf der Schraube ist mit einem Gewinde versehen (Kopfgewinde), das dazu ausgelegt ist, das das Durchgangsloch umgebende Material des Inlays umzuformen, um eine Gewindeverbindung zu bilden. Für eine Mehrzahl von Durchgangslöchern der Knochenplatte kann das System eine Mehrzahl von Knochenschrauben umfassen. Da die Gewindeverbindung erst beim Eindrehen der Knochenschraube gebildet wird, kann die Knochenschraube unter verschiedenen Winkeln (winkelvariabel) mit der Knochenplatte verbunden werden.

Die Knochenplatte kann mit weiteren Merkmalen fortgebildet werden, die im Zusammenhang des erfindungsgemäßen Verfahrens beschrieben sind. Das Verfahren kann mit weiteren Merkmalen fortgebildet werden, die im Zusammenhang der erfindungsgemäßen Knochenplatte beschrieben sind. Die Erfindung betrifft außerdem eine Knochenplatte, die mit dem erfindungsgemäßen Verfahren herstellbar ist.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine erfindungsgemäße Knochenplatte;
- Fig. 2:: eine Knochenschraube eines erfindungsgemäßen Knochenplattensystems;
- Fig. 3:: ein Verwendungsbeispiel eines erfindungsgemäßen Knochenplattensystems;
- Fig. 4 bis 6:: einen erfindungsgemäßen Verfahrensablauf;
- Fig. 7:: die Ansicht gemäß Fig. 6 bei einer anderen Ausführungsform der Erfindung.

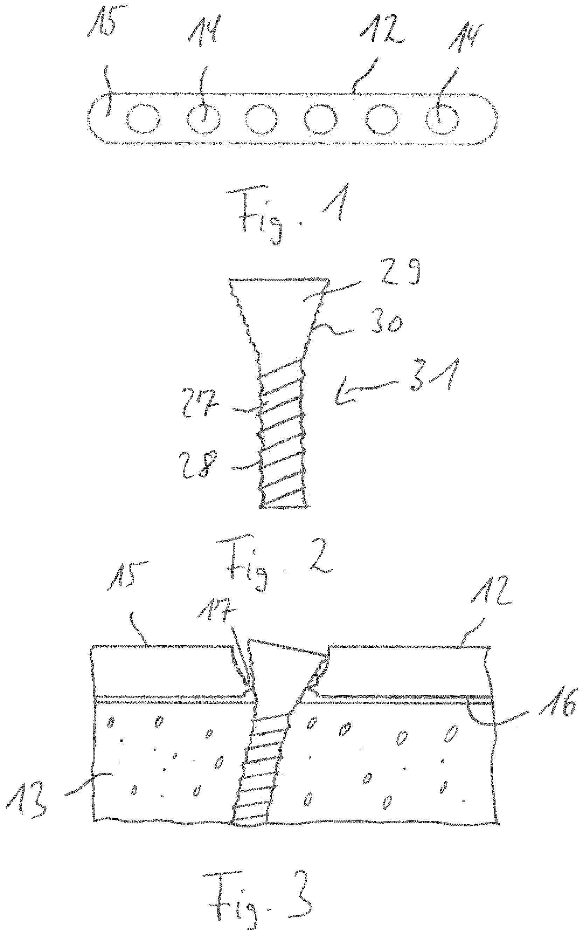

Eine erfindungsgemäße Knochenplatte 12 hat gemäß Fig. 1 sechs Durchgangslöcher 14, die sich von einer Oberseite 15 der Knochenplatte bis zu einer Unterseite 16 der Knochenplatte erstrecken. In jedem Durchgangsloch 14 ist eine Materiallippe 17 ausgebildet, die von der Wand des Durchgangslochs 14 in Richtung des Zentrums des Durchgangslochs 14 vorspringt. Die Materiallippe 17 ist eine durchgehende Materiallippe, die sich ohne Unterbrechung über den gesamten Umfang des Durchgangslochs 14 erstreckt. Die Materiallippe 17 ist im unteren Drittel des Durchgangslochs 14 angeordnet, so dass oberhalb der Materiallippe 17 Raum für die Aufnahme eines Schraubenkopfs bleibt.

Die Knochenplatte ist dazu bestimmt, mit der Unterseite 16 auf einen Knochen 13 aufgelegt zu werden, so dass die Knochenplatte sich über eine Bruchstelle des Knochens hinweg erstreckt. Nachdem der Knochen in die richtige Stellung reponiert ist, wird die Knochenplatte mit den Knochenfragmenten verbunden. Der Knochen ist dann in dieser Stellung fixiert und kann ausheilen.

Zum Verbinden der Knochenplatte mit dem Knochen werden Knochenschrauben 31 gemäß der beispielhaften Darstellung in Fig. 2 verwendet. Die Knochenschrauben 31 haben einen Schaft 27, der mit einem Knochengewinde 28 versehen ist, und einen Schraubenkopf 29, dessen Außenfläche ein Kopfgewinde 30 mit kleinerer Steigung aufweist. Die Knochenschraube wird in den Knochen eingedreht, bis der Schraubenkopf 29 in das Durchgangsloch 14 eindringt. Dabei kann der Winkel, den die Knochenschraube mit der Achse des Durchgangslochs einschließt, zwischen etwa 0° und 15° frei gewählt werden. Wenn die Knochenschraube nun weiter eingedreht wird, kommt das Kopfgewinde 30 der Knochenschraube 31 in Eingriff mit der Materiallippe 17. Die Materiallippe 17 wird umgeformt und es wird eine Gewindeverbindung zwischen dem Kopf 29 der Knochenschraube 31 und der Materiallippe 17 gebildet.

Bei der erfindungsgemäßen Knochenplatte 12 sind die sechs Durchgangslöcher 14 jeweils in einem Inlay 19 ausgebildet. Das Inlay besteht aus Reintitan Grade 1 oder Grade 0 und damit aus einem Material vergleichsweise geringer Härte. Die Inlays sind eingesetzt in passende Ausnehmungen 33 einer tragenden Struktur 20 der Knochenplatte. Die tragende Struktur 20 besteht aus Reintitan Grade 4 oder aus der Titanlegierung TiAl6V4.

Die Knochenschraube 31 besteht aus der Titanlegierung TiAl6V4 und damit aus einem härteren Material als das Inlay 19. Kommt der Kopf 29 der Knochenschraube in Eingriff mit der Materiallippe 17, verformt sich die Materiallippe 17 und es entsteht eine Gewindeverbindung zwischen dem Kopfgewinde 30 und der Materiallippe 17. Die Knochenschraube 31 wird dadurch winkelstabil mit der Knochenplatte verbunden.

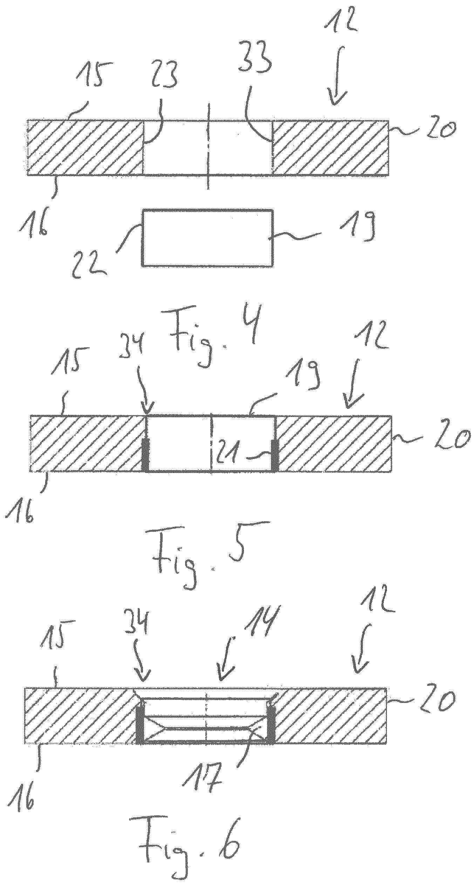

In den Fig. 4 bis 6 ist das erfindungsgemäße Verfahren zum Herstellen der Knochenplatte 12 am Beispiel eines der Durchgangslöcher 14 dargestellt. Eine tragende Struktur 20 der Knochenplatte besteht aus Reintitan Grade 4 und hat für jedes der Durchgangslöcher 14 eine konzentrisch zu dem späteren Durchgangsloch 14 angeordnete Ausnehmung 33. In die Ausnehmung 33 wird ein als Vollkörper ausgebildetes Inlay 19 aus Reintitan Grade 1 eingesetzt, so dass die Mantelfläche 22 des Inlays 19 flächig auf der Innenmantelfläche 23 der Ausnehmung 33 aufliegt. Der Bereich, in dem die Mantelfläche des Inlays 19 und die Innenmantelfläche der Ausnehmung 33 aufeinander liegen, wird als Kontaktfläche bezeichnet. Sowohl das Inlay 19 als auch die Ausnehmung 33 haben eine leichte Konusform mit einem Konuswinkel von etwa 1°, die sich von der Unterseite 16 zur Oberseite 15 hin verjüngt. An der Oberseite 15 und an der Unterseite 16 der Knochenplatte 12 schließen die Oberflächen des Inlays 19 und der tragenden Struktur 20 bündig miteinander ab.

Von der Unterseite 16 der Knochenplatte 12 wird durch Laserschweißen eine Schweißnaht 21 erzeugt, die bis in eine Tiefe von etwa 2,5 mm in das Material vordringt. Die Knochenplatte hat eine Materialstärke von 3,8 mm, so dass die Schweißnaht 21 sich über etwas mehr als die Hälfte der Materialstärke erstreckt. Die Schweißnaht 21 erstreckt sich also nicht über die gesamte Höhe der Kontaktfläche, sondern nur über einen Höhenabschnitt der Kontaktfläche.

Der Zustand, in dem das noch als Vollkörper ausgebildete Inlay 19 auf diese Weise mit der tragenden Struktur 20 verschweißt ist, ist in Fig. 5 dargestellt. Es schließt sich eine mechanische Bearbeitung durch Fräsen an, mit der durch das Inlay 19 hindurch ein Durchgangsloch 14 erzeugt wird. Im unteren Bereich des Durchgangslochs 14 bleibt die Materiallippe 17 stehen, die dafür bestimmt ist, später durch den Kopf 29 der Knochenschraube 31 umgeformt zu werden.

Mit der mechanischen Bearbeitung wird insbesondere Material in dem Übergangsbereich 34 abgetragen, in dem das Inlay 19 und die tragende Struktur aneinander liegen, ohne miteinander verschweißt zu sein. Beim Abtragen des Materials wird der Übergangsbereich 34 mechanisch behandelt, so dass die Naht zwischen dem Inlay 19 und der tragenden Struktur 20 verschlossen wird und sich in dem Übergangsbereich 34 eine geschlossene Oberfläche ergibt.

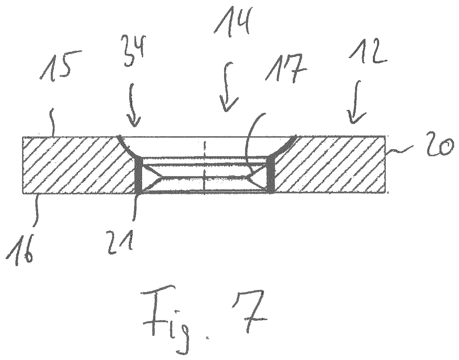

In Fig. 7 ist eine Ausführungsform der Erfindung dargestellt, bei der mit der mechanischen Bearbeitung tiefer in das Material der tragenden Struktur 20 und des Inlays 19 eingegriffen wird, so dass mit der mechanischen Bearbeitung bis in den Bereich der Schweißnaht 21 vorgedrungen wird. Bei dieser Ausführungsform wird der geschlossene Übergang zwischen dem Inlay 19 und der tragenden Struktur 20 durch die Schweißnaht 21 gebildet.

## Patentansprüche

1. Verfahren zum Herstellen einer Knochenplatte, bei dem ein Inlay (19) in eine Ausnehmung (33) einer tragenden Struktur (20) eingesetzt wird, so dass eine Mantelfläche (22) des Inlays (19) an einer Innenmantelfläche (23) der Ausnehmung (33) anliegt, und bei dem das Inlay (19) und die tragende Struktur (20) mit einer Schweißnaht (21) verbunden werden, so dass die Eindringtiefe der Schweißnaht (21) kleiner ist als die Materialstärke im Bereich der Schweißnaht (21).

2. Verfahren nach Anspruch 1, **dadurch gekennzeichnet, dass** ein der Schweißnaht (21) gegenüberliegender Übergangsbereich (34) zwischen der tragenden Struktur (20) und dem Inlay (19) mechanisch bearbeitet wird.

3. Verfahren nach Anspruch 2, **dadurch gekennzeichnet, dass** in dem Übergangsbereich (34) eine geschlossene Oberfläche erzeugt wird.

4. Verfahren nach Anspruch 2 oder 3, **dadurch gekennzeichnet, dass** in dem Übergangsbereich (34) Material von dem Inlay (19) und/oder von der tragenden Struktur (20) abgetragen wird, so dass die Materialstärke im Bereich der Kontaktfläche zwischen dem Inlay (19) und der tragenden Struktur (20) vermindert wird.

5. Verfahren nach einem der Ansprüche 2 bis 4, **dadurch gekennzeichnet, dass** mit der mechanischen Bearbeitung bis zu der Schweißnaht (21) vorgedrungen wird.

6. Verfahren nach einem der Ansprüche 1 bis 5, **dadurch gekennzeichnet, dass** nach dem Erzeugen der Schweißnaht (21) ein Durchgangsloch (14) und/oder eine von einer Lochwand des Durchgangslochs (14) vorspringende Materiallippe (17) in dem Inlay (19) erzeugt werden.

7. Verfahren nach einem der Ansprüche 1 bis 6, **dadurch gekennzeichnet, dass** die Schweißnaht (21) von einer Unterseite (16) der Knochenplatte ausgeführt ist.

8. Verfahren nach einem der Ansprüche 1 bis 7, **dadurch gekennzeichnet, dass** das Material des Inlays (19) eine geringere Härte hat als das Material der tragenden Struktur (20) .

9. Verfahren nach einem der Ansprüche 1 bis 8, **dadurch gekennzeichnet, dass** die Knochenplatte eine Dicke von mehr als 2 mm, vorzugsweise von mehr als 3 mm hat.

10. Knochenplatte mit einem Durchgangsloch (14), das sich zwischen einer Oberseite (15) und einer Unterseite (16) der Knochenplatte erstreckt, wobei eine tragende Struktur (20) der Knochenplatte aus einem ersten Material gebildet ist und wobei das Durchgangsloch (14) in einem Inlay (19) aus einem zweiten Material ausgebildet ist, wobei die tragende Struktur (20) und das Inlay (19) durch eine Schweißnaht (21) miteinander verbunden sind und wobei ein der Schweißnaht (21) gegenüberliegender Übergangsbereich (34) zwischen der tragenden Struktur (20) und dem Inlay (19) mechanisch bearbeitet ist.

11. System aus einer Knochenplatte gemäß Anspruch 10 und einer Knochenschraube (31), wobei der Kopf (29) der Schraube (31) mit einem Gewinde (29) versehen ist, das dazu ausgelegt ist, das das Durchgangsloch (14) umgebende Material des Inlays (19) umzuformen, um eine Gewindeverbindung zu bilden.
